# EUROPÄISCHE PATENTSCHRIFT

(11) **EP 0 929 579 B1**
(45) Veröffentlichungstag und Bekanntmachung des Hinweises auf die Patenterteilung: **08.01.2003**
(21) Anmeldenummer: 97911202.6
(22) Anmeldetag: 09.10.1997
(51) Int. Cl.: C07K 16/38, C07K 16/40, C07K 14/705, C07K 14/81, C12N 5/20, C12N 9/64, G01N 33/574

(54) **MONOKLONALE ANTIKÖRPER GEGEN EINEN KOMPLEX AUS HUMANEM ACT UND EINER SERINPROTEASE**
MONOCLONAL ANTIBODIES AGAINST A HUMAN ACT AND SERINE PROTEASE COMPLEX
ANTICORPS MONOCLONAUX CONTRE UN COMPLEXE D'ACT HUMAIN ET D'UNE SERINE-PROTEASE

(30) Priorität: 09.10.1996 DE 19641560
(43) Veröffentlichungstag der Anmeldung: 21.07.1999
(73) Patentinhaber: Roche Diagnostics GmbH, 68298 Mannheim (DE)
(72) Erfinder: HÜBNER-PARAJSZ, Christa, D-82327 Tutzing (DE); SCHETTERS, Hartmut, D-82377 Penzberg (DE); KIENTSCH-ENGEL, Rosemarie, D-82340 Feldafing (DE); MEIER, Thomas, D-80634 München (DE); KAUFMANN, Martin, D-82362 Weilheim (DE); GALLUSSER, Andreas, D-82377 Penzberg (DE); DEEG, Rolf, D-82347 Bernried (DE)
(86) Internationale Anmeldenummer: EP9705556
(87) Internationale Veröffentlichungsnummer: WO98015580

(56) Entgegenhaltungen:
- EP-A- 0 635 575
- WO-A-92/01936
- WO-A-95/18381
- DE-A- 4 322 342
- WU ET AL.: "CORRELATION OF SERUM CONCENTRATIONS OF PSA-ACT COMPLEX WITH TOTAL PSA IN RANDOM AND SERIAL SPECIMENS FROM PATIENTS WITH BPH AND PROSTATE CANCER" JOURNAL OF CLINICAL LABORATORY ANALYSIS, Bd. 9, 1995, Seiten 15-24, XP002054744
- JOSLIN ET AL.: "CROSS-COMPETITION FOR BINDING OF ALPHA1-ANTITRYPSIN (ALPHA1 AT)-ELASTASE COMPLEXES TO THE SERPIN-ENZYME COMPLEX RECEPTOR BY OTHER SERPIN-ENZYME COMPLEXES AND BY PROTEOLYTICALLY MODIFIED ALPHA1 AT" JOURNAL OF BIOLOGICAL CHEMISTRY, Bd. 268, Nr. 3, 1993, Seiten 1886-1893, XP002054745 in der Anmeldung erwähnt
- PERLMUTTER ET AL.: "IDENTIFICATION OF A SERPIN-ENZYM COMPLEX RECEPTOR ON HUMAN HEPATOMA CELLS AND HUMAN MONOCYTES" PROC.NATL.ACAD.SCI., Bd. 87, 1990, Seiten 3753-3757, XP002054746 in der Anmeldung erwähnt

## Beschreibung

Gegenstand der vorliegenden Erfindung sind monoklonale Antikörper (MAK), die spezifisch einen Komplex aus α1-Antichymotrypsin (ACT) und einer Serinprotease, insbesondere Prostata-spezifisches Antigen (PSA) binden und im wesentlichen keine Kreuzreaktivität zu nicht komplexiertem ACT und nicht komplexierten Serinproteasen aufweisen. Diese monoklonalen Antikörper können zum Nachweis von ACT-Serinproteasen-Komplexen insbesondere von PSA-ACT verwendet werden.

Das Prostata-spezifische Antigen ist ein Glykoprotein mit einem Molekulargewicht von 33 kDa. Es wird in den Prostataepithelzellen gebildet und ist ein Bestandteil der Samenflüssigkeit. PSA hat die enzymatische Aktivität einer neutralen Serinprotease.

Die Hauptfunktion des PSA besteht in der Spaltung der Seminogeline I und II sowie des Fibronektins, gelartiger Proteine, die als wesentliche Komponenten des Ejakulats die Beweglichkeit der Spermien blockieren. Durch die Hydrolyse dieser Proteine bewirkt PSA die Verflüssigung des Samenkoagulums und ermöglicht so die Mobilität der Spermien.

Das enzymatisch aktive PSA wird im Serum durch verschiedene Inhibitoren, sog. Serpine (= Serinproteaseninhibitoren) durch Bildung von kovalenten Komplexen inaktiviert. Die Hauptmenge des immunologisch nachweisbaren PSA ist im Serum an α1-Antichymotrypsin (zu 60-95 % ) gebunden. Weitere Komplexe werden mit α2-Makroglobulin, α1-Antitrypsin, Inter-α- Trypsininhibitor und Protein C-Inhibitor gebildet. Daneben kommt noch ein enzymatisch inaktives PSA vor, das nicht mehr mit Serpinen komplexiert. α1-Antichymotrypsin ist ein Glykoprotein mit einem Molekulargewicht von ca. 69 kDa und einem Kohlenhydratanteil. Als eine der Hauptinhibitoren in der akuten Phase spielt ACT eine wichtige Rolle bei der Kontrolle von Entzündungen. ACT bildet auch Komplexe mit Chymotrypsin,Cathepsin G und glandulärem Kallikrein hK2. ACT kommt im Humanserum in 10.000-fach höherer Konzentration vor als PSA ( auf molarer Basis ).

Der PSA-ACT-Komplex ist neben freiem PSA die Hauptform des immunologisch nachweisbaren Gesamt-PSA im Serum. Prostatakrebs führt in vielen Fällen zu einer Erhöhung des Serum-PSA-Spiegels. Da sich jedoch auch bei benigner Prostatahyperplasie leicht erhöhte PSA-Serumwerte finden, ist PSA vor allem im niedrigen Konzentrationsbereich kein krebsspezifischer Marker. Bei den bisher erhältlichen Screeningtests auf das mögliche Vorliegen eines Prostatacarcinoms in einem Patienten handelte es sich immer um Tests zum Nachweis von totalem PSA. Da PSA im Serum von männlichen Personen normalerweise in sehr niedrigen Konzentrationen vorkommt, muß in einem solchen Test ein sogenannter "Cut-Off" definiert werden. PSA-Werte die über diesem Cut-Off liegen, werden als Hinweis auf das Vorliegen von Prostatacarcinom gewertet. Da die PSA-Konzentration mit zunehmendem Alter der Patienten steigt, wurden bisher bei dem Test zum Nachweis des totalen PSA Cut-Off Werte von 4 bis 6 ng/ml benutzt. Das hatte zur Folge, daß einige Patienten, die ein Prostatacarcinom im Frühstadium besaßen, nicht in diesen Screeningtesten erfaßt wurden.

Bereits in der japanischen Offenlegungsschrift 62-46263 wurde gefunden, daß bei Patienten mit malignem Prostatatumor erhöhte Werte an komplexiertem PSA, im Vergleich zu Patienten mit benigner Prostatahyperplasie auftreten. In dieser Offenlegungsschrift wurde ein Immunoassay beschrieben, der durch die Kombination eines Antikörpers gegen γ-Seminoprotein (γ-Seminoprotein ist identisch mit PSA; siehe Schaller et al., Eur. J. Biochem. 170, 1987, 111-120 und Nakamura, Cancer 74, 1994, 1655-1659) und eines Antikörpers gegen α₁-Antitrypsin nachweist.

In der WO 92/01936 wird eine Methode zum Nachweis von PSA-ACT beschrieben, bei der eine Kombination des Antikörpers 2E9, der unkomplexiertes PSA und auch PSA im Komplex mit ACT bindet, und eines Antikörpers gegen ACT eingesetzt wird.

Es existieren weiterhin diagnostische Tests zum Nachweis von freiem, nicht komplexiertem PSA und Gesamt-PSA, d.h. der Summe aus freiem und komplexiertem PSA. Alle diese Tests enthalten Antikörper, die im Falle des Nachweises von freiem PSA nur PSA in nicht komplexierter Form oder im Falle des Nachweises von Gesamt-PSA PSA in komplexierter und freier Form erkennen.

Der Nachweis von ACT in komplexierter Form mit Serinproteinasen, insbesondere der Nachweis von PSA-ACT gelang, wie oben beschrieben, bisher nur mit Hilfe eines Sandwich-Testes mit zwei Antikörpern, wovon der eine Antikörper gegen PSA und der andere Antikörper gegen ACT gerichtet war. Da ACT in Humanserum in ca. 10.000-fachem Überschuß gegenüber PSA, und somit auch dem Komplex aus PSA und ACT vorkommt, ist es nicht auszuschließen, daß durch diesen hohen Überschuß an ACT der Test negativ gestört wird. Insbesondere ist es unerläßlich, bei diesen bisher bekannten Tests zum Nachweis von PSA-ACT mindestens einen Waschschritt zum Entfernen von überschüssigem ACT vor der Zugabe des ACT-spezifischen Antikörpers in das Testprozedere einzufügen. Eine Einschrittestfiihrung, die bei vielen automatisierten diagnostischen Tests angestrebt wird, ist damit nicht möglich.

In der WO 95/18381 wird ein Verfahren zum Nachweis von PSA beschrieben, bei dem freies PSA und der Gesamtgehalt an PSA (PSA total) mittels eines Sandwich-Tests nachgewiesen wird. Der spezifische Nachweis von PSA-ACT ist nicht möglich, da nur Antikörper verwendet werden, die entweder für freies PSA spezifisch sind oder mit freiem PSA und dem PSA-ACT-Komplex reagieren.

Aufgabe der vorliegenden Erfindung war es daher, einen verbesserten Test zum Nachweis von PSA-Serinproteasen, insbesondere PSA-ACT bereitzustellen. der möglichst nicht durch die im Serum vorkommenden hohen ACT-Konzentrationen gestört wird, und der es erlaubt, ein möglichst sensitives Screening zum Nachweis eines Prostatacarcinoms durchzuführen.

Gelöst wurde die Aufgabe durch einen monoklonalen Antikörper gegen einen Komplex aus humanem ACT und einer Serinprotease der im wesentlichen keine Kreuzreaktivität mit freiem, nicht komplexiertem humanem ACT und freien, nicht komplexierten Serinproteasen aufweist.

Insbesondere wurde die Aufgabe gelöst durch einen MAK gegen einen Komplex aus ACT und einer Serinprotease, der im wesentlichen keine Kreuzreaktivität mit freiem humanem ACT und freien Serinproteasen aufweist, und der eine wesentlich höhere Affinität und Spezifität zu PSA-ACT als zu anderen Serinprotease-ACT-Komplexen, insbesondere zu Chymotrypsin-ACT und Cathepsin-G-ACT aufweist.

Der monoklonale Antikörper kann in allen dem Fachmann geläufigen Tests zum Nachweis eines Proteins verwendet werden. Bei der bevorzugten Testführung mit zwei Antikörpern (Sandwich-Test) kann der Test einstufig, also ohne zusätzlichen Waschschritt zum Entfernen des überschüssigen ACT durchgeführt werden. Besonders für Screeningtests, bei denen eine Vielzahl von Proben in möglichst rascher Folge getestet werden sollen, ist dies ein entscheidender Fortschritt gegenüber den bisher möglichen Tests, die alle einen Waschschritt zum Entfernen des überschüssigen ACT enthielten.

Die erfindungsgemäßen monolonalen Antikörper gegen einen Komplex aus humanem ACT und einer Serinprotease besitzen im wesentlichen keine Kreuzreaktivität mit freiem humanem ACT und freien Serinproteasen. Unter im wesentlichen keine Kreuzreaktivität wird eine Kreuzreaktivität verstanden, die im Test zum Nachweis des Komplexes ACT-Serinprotease keine Beeinflussungen durch freies ACT oder freie Serinprotease bewirkt. Wie hoch die noch tolerierte Kreuzreaktivität gegen einzelne Komponenten sein kann, hängt davon ab, in welcher Konzentration diese Komponente im Humanserum vorkommen kann. Da ACT in einem sehr großen Überschuß vorkommt, darf in diesem Fall die Kreuzreaktivität nur verschwindend gering, d.h. weit unter 1% liegen. Im Falle der erfindungsgemäßen monoklonalen Antikörper konnte mit den zur Verfügung stehenden Methoden keine Kreuzreaktivität nachgewiesen werden. Zum Nachweis der Kreuzreaktivität wurde das BIAcore®-System der Firma Pharmacia benutzt. Antikörper mit einer Affinitätskonstante von weniger als 10⁵ l/mol zu den getesteten Substanzen zeigten keine nennenswerte Bindung und damit keine nachweisbare Kreuzreaktivität in diesem System.

Die erfindungsgemäßen monoklonalen Antikörper zeigten gegenüber nicht komplexiertem PSA, Chymotrypsin und Cathepsin G im BIAcore® keine Kreuzreaktivität. Um die Kreuzreaktion mit allen in humanem Serum vorkommenden, möglicherweise störenden Substanzen zu überprüfen, wurde für diesen Fall dem Screening-Test humanes Serum zugesetzt. Damit keine ACT-PSA-Komplexe vorhanden sind, wurde ein humanes, weibliches Serum benutzt. Die erfindungsgemäßen monoklonalen Antikörper zeigen keine nachweisbare Kreuzreaktivität mit anderen in diesem Serum vorkommenden Bestandteilen.

Da PSA-ACT den klinisch relevantesten Serinproteasen-ACT-Komplex darstellt, besitzen die erfindungsgemäßen monoklonalen Antikörper insbesondere eine höhere Affinität und Spezißtät zu PSA-ACT als zu den anderen Serinprotease-ACT-Komplexen. Bevorzugt ist die Affinität zu PSA-ACT mindestens 10fach höher, besonders bevorzugt 50fach oder höher. Mittels dieser spezifischen monoklonalen Antikörper gegen PSA-ACT ist es möglich, einen Einschrittest zum Nachweis von PSA-ACT zu gestalten, der durch das nicht komplexierte PSA undACT nicht oder nicht klinisch signifikant gestört wird.

Die erfindungsgemäßen monoklonalen Antikörper besitzen bevorzugt eine Affinität zu PSA-ACT von mindestens 10⁷ l/mol, besonders bevorzugt von mindestens 10⁹ l/mol. Bei einem erfindungsgemäßen monoklonalen Antikörper konnte sogar eine für monoklonale Antikörper ungewöhnlich hohe Affinität von 10¹⁰l/mol festgestellt werden. Solche hochaffinen monoklonalen Antikörper gegen PSA-ACT sind hervorragend für Einschritteste geeignet, bei denen gewöhnlich eine relativ kurze Inkubation der Probe mit dem monoklonalen Antikörper erfolgt. Die Bindung dieses hochaffinen monoklonalen Antikörper an PSA-ACT erfolgt sehr rasch.

Die erfindungsgemäßen monoklonalen Antikörper können allen möglichen Ig-Klassen angehören. Bevorzugt gehören die monoklonalen Antikörper der IgG1-Klasse an. Die Kopplung von weiteren Komponenten wie beispielsweise Bindepartner zur Bindung des Antikörpers an eine Festphase bei heterogenen Immunoassays, oder beispielsweise Markierungen wie Enzyme usw. kann bevorzugt an IgG1-Antikörpem erfolgen. Auch die Spaltung zu Antikörper-Fragmenten ist bei der IgG1-Klasse unproblematisch.

Erfindungsgemäß wird unter der Bezeichnung monoklonaler Antikörper sowohl der vollständige Antikörper als auch alle bei Immuntests und sonstigen Verwendungen gängigen Fragmente davon, wie F(ab')₂ und Fab-Fragmente verstanden. Umfaßt sind auch solche Antikörper, die durch Veränderung der monoklonalen Antikörper hergestellt wurden, solange die Antigenbindeeigenschaft nicht entscheidend beeinflußt wurde. Beispielsweise können durch gentechnologische Maßnahmen Teile der normalerweise in Mäusen hergestellten monoklonalen Antikörper durch entsprechende humane Antikörpersequenzen ersetzt werden, um unspezifische Bindungen im Immunoassay zu minimieren. Verfahren zur Herstellung solcher chimären monoklonalen Antikörper sind dem Fachmann beispielsweise aus Antibody Engineering, J. Mc Cafferty, H.R. Hoogenboom und D.J. Chiswell, The Practical Approach Series, Series Editor: B.D. Hames, Oxford University Press, 1996 bekannt.

Die erfindungsgemäßen monoklonalen Antikörper können beispielsweise aus den Zellinien MAK<PSA-ACT>M 4.6.374, MAK<PSA-ACT>M 4.3.2 und MAK<PSA-ACT>M 6.13.64, hinterlegt am 19.9.1996.bei der DSM (Deutsche Sammlung von Mikroorganismen und Zellkulturen GmbH, Mascheroder Weg 1b, D-38124 Braunschweig) hergestellt werden. (MAK<PSA-ACT>M 4.6.374 = DSM ACC 2281; MAK<PSA-ACT>M 6.13.64 = DSM ACC 2282; MAK<PSA-ACT>M 4.3.2 = DSM ACC 2283).

Gegenstand der Erfindung sind weiterhin Antikörper bevorzugt monoklonale Antikörper, die in äquivalenter Weise an Serinproteasen-ACT-Komplexe binden, wie die monoklonalen Antikörper 4.6.374, 4.3.2 und 6.13.64. Unter in äquivalenter Weise binden wird verstanden, daß diese Antikörper das gleiche Epitop wie die hinterlegten monoklonalen Antikörper erkennen. Dies kann beispielsweise durch Multi Binding-Versuche am BIAcore® festgestellt werden.

Die erfindungsgemäßen monoklonalen Antikörper können in an sich bekannter Weise durch Immunisierung mit PSA-ACT humanen Ursprungs in geeigneten Versuchstieren und anschließender Fusion der Milzzellen der immunisierten Tiere mit Myelomzellen hergestellt werden. Die Ausbeute an Serinproteasen-ACT spezifischen monoklonalen Antikörper war jedoch sehr gering. Durch die Verwendung von ausschließlich weiblichen Versuchstieren konnte die Ausbeute an PSA-Serinprotease-spezifischen Antikörpern erhöht werden. Selbst hier besaßen noch cirka 70% der monoklonalen Antikörper eine hohe Kreuzreaktivität zu ACT und ca. 30% eine hohe Kreuzreaktivität zu PSA. Weit unter 1% der insgesamt erhaltenen Antikörper besaßen die erforderliche Spezifität für den Serinprotease-Inhibitor-Komplex bzw. den PSA-ACT-Komplex.
Neben Milzzellen als Lymphozyten-Quelle können auch PBL ( Periphere Blut Lymphozyten ) oder Lymphknoten-Zellen von immunisierten Tieren (bevorzugt der Maus und der Ratte) verwendet werden.

Alternativ können auch Lymphozyten (PBL, Milzzellen, Lymphknoten-Zellen) von humanen Spendern (wie Prostata-Tumor-Patienten, laktierenden Frauen, Patienten mit PSA-sezernierenden Zellen / Geweben), die Antikörper oder Auto-Antikörper gegen PSA-ACT entwickelt haben, immortalisiert werden. Solche anti-PSA-ACT-produzierende Lymphozyten können entweder durch Fusion mit einer humanen Myelomlinie oder durch EBV ( Epstein Barr - Virus ) -Transformation zu Antikörper-produzierenden Hybridoma-Zellen immortalisiert werden (Monodonal Antibody and Immunosensor Technology, A. M. Campbell, Elsevier Verlag 1991; Monoklonale Antikörper, J.H. Peters, H. Baumgarten, Springer Verlag 1990; Monodonal Antibody Production Techniques and Applications, ed. Lawrence B. Schook, Marcel Dekker Verlag 1987).

Ein weiterer Gegenstand der Erfindung ist die Verwendung der erfindungsgemäßen monoklonalen Antikörper zum Nachweis von Serinprotease-ACT-Komplexen, insbesondere von PSA-ACT in Proben, bevorzugt humanen Proben wie beispielsweise Plasma, Serum, Blut, Seminalflüssigkeit, Prostata-Flüssigkeit, Seminal-Vesikel-Flüssigkeit, Speichel, Liquor, Frauen-Milch, Zysten, Gewebe-Homogenate, Gewebeschnitte, Biopsiematerial.

Da die erfindungsgemäßen monoklonalen Antikörper spezifisch den Komplex aus Serinproteasen und humanem ACT erkennen, wobei davon auszugehen ist, daß alle erfindungsgemäßen MAK ein Epitop erkennen, das nur in diesem Komplex, nicht jedoch in freien Serinproteasen und freiem humanem ACT vorkommt, ist es möglich, alle gängigen Testkonfigurationen, die zum Nachweis eines Proteins geeignet sind, zu verwenden. Der Fachmann ist daher nicht mehr wie bisher ausschließlich auf einen Zweischritt-Sandwichassay beschränkt, der zudem vor der Inkubation des humanen ACT-spezifischen Antikörpers einen Waschschritt unbedingt beinhalten mußte.

Gegenstand der Erfindung ist daher weiterhin ein Verfahren zur Bestimmung eines Komplexes aus humanem ACT und Serinproteasen durch Inkubation der Probe mit mindestens einem erfindungsgemäßen monoklonalen Antikörper. Alle gängigen, dem Fachmann geläufigen Methoden zum Nachweis eines Proteins, wie beispielsweise kompetitive Teste nach dem IEMA-Prinzip oder direkte Teste wie Sandwich-Teste, sind geeignet. Neben heterogenen Testen bei denen die Assaykomponenten an eine Festphase gekoppelt sind und eine Trennung von fester und flüssiger Phase erfolgt, können auch homogene Teste, die zum Nachweis eines Proteins geeignet sind, verwendet werden. Beispiele hierfür sind nephelometrische oder turbidimetrische Tests wie Latexagglutinationstests oder TINIA (turbidimetrische Inhibitions-Immunoassays). Neben den sogenannten Naßtesten, bei denen die Testreagenzien in flüssiger Phase vorliegen, können auch alle gängigen Trockentestformate, die zum Nachweis eines Proteins geeignet sind, verwendet werden. Bei diesen Trockentests oder Teststreifen sind die Testkomponenten auf einem Träger aufgebracht. Solche Trockentests sind beispielsweise beschrieben in EP-A 0 186 799.

Ein weiterer Gegenstand der Erfindung ist ein Test zum Nachweis von PSA-ACT durch Inkubation der Probe mit einem erfindungsgemäßen monoklonalen Antikörper, der eine höhere Affinität zu PSA-ACT als zu den anderen Serinproteasen-ACT-Komplexen aufweist. Bevorzugt besitzt dieser MAK eine mindestens 10- besonders bevorzugt eine mindestens 50fache höhere Affinität zu PSA-ACT.

Bei Verwendung einer Kombination aus mehreren Antikörpern im Test, von denen einer ein erfindungsgemäßer monoklonaler Antikörper ist, ist es möglich, PSA-ACT spezifische Teste auch bei Verwendung eines monoklonalen Antikörpers, der alle Serinproteasen-ACT-Komplexe etwa gleich gut erkennt, zu gestalten. Ein Gegenstand der Erfindung ist daher ein Verfahren zur Bestimmung von PSA-ACT durch Inkubation der Probe mit mindestens einem erfindungsgemäßen monoklonalen Antikörper und einem Antikörper, der gegen PSA gerichtet ist. Solche PSA spezifischen Antikörper sind bekannt und werden bereits seit 1985 in diagnostischen Tests zum Nachweis von PSA bzw. γ-Seminoprotein eingesetzt. Durch die Kombination dieser beiden Antikörper wird PSA-ACT spezifisch nachgewiesen. Welcher der beiden Antikörper bei dem in diesem Fall üblichen Sandwich-Test in markierter Form oder an die Festphase gebunden vorliegt, ist gleich.

Anstelle des monoklonalen Antikörpers spezifisch für den Komplex aus ACT und einer Serinprotease, insbesondere des ACT-PSA-Komplexes ist es auch möglich, einen Rezeptor für PSA-ACT in Kombination mit dem Antikörper gegen PSA einzusetzen. PSA gehört zur Familie der Kallikreine, einer Proteasegruppierung, die hohe Homologien untereinander aufweisen. Sie werden von Inhibitoren, den sogenannten Serpinen (Serine Protease Inhibitors) gebunden, zu denen ACT gehört. Diese PSA-Serpin-Komplexe besitzen ein Epitop, ein sogenanntes Neo-Epitop, das auf den freien Inhibitoren, die keine Protease gebunden haben, nicht vorhanden ist. Die Eliminierung dieser PSA-ACT-Komplexe bzw. allgemein der Kallikrein-Serpin-Komplexe aus der Blutzirkulation erfolgt über Rezeptoren, die dieses Neo-Epitop erkennen. Das Neo-Epitop ist beispielsweise beschrieben in Perlmutter et al., J. Biol. Chem. 265, No. 28, 16713-16716, 1990; Perlmutter et al., Proc. Natl. Acac. Sci. USA, 87, 3753-3757, 1990 und Joslin et al, J. Biol. Chem. 268, No. 3, 1886-1893, 1993. Die Rezeptoren für PSA-ACT bzw. Kallikrein-Serpine wurden bei Hep G2-Zellen nachgewiesen (Joslin et al. 1993) und können hieraus isoliert werden. Ein weiterer Gegenstand der Erfindung ist daher ein Verfahren zur Bestimmung von PSA-ACT durch Inkubation der Probe mit einem Rezeptor von PSA-ACT, der das oben näher beschriebene Neo-Epitop bindet, und einem Antikörper bevorzugt einem monoklonalen Antikörper, der gegen PSA gerichtet ist. Ob der Rezeptor oder der Antikörper in markierter Form oder an die Festphase gebunden vorliegt, ist dabei gleich.

Zur Herstellung der Serpin-Protease-Rezeptoren werden zunächst Zellen gezüchtet, die den gewünschen Rezeptor exprimieren. Der Nachweis des Rezeptors erfolgt beispielsweise mit einem Zell ELISA wie von Hashemi et al., J. Lab. Clin. Med. 109 (1987), 434-440 beschrieben. Hierzu werden Hep G2 Zellen in Flachbodenkulturgefäßen (Costar) in einer Dichte von 2 x 10⁴ Zellen/ml ausgesäht. Als Kulturmedium wird D'MEM mit 10% fötalem Kälberserum (FCS) genutzt. Die Hep G2-Zellen werden bis zu 70 % Gesamtmenge wachsen und anschließend unfixiert gelassen. ACT-PSA (Scripps Laboratories) wird bis zu einer Endkonzentration von 10µg/ml in D'MEM/1 % RSA zugefügt und zum Komplexieren mit dem Rezeptor für 10 Minuten bei 4°C stehengelassen. Nach zweimaligem Waschen mit D'MEM/1% RSA werden die Zellen mit einem anti PSA monoklonalen Antikörper, der mit Peroxidase markiert ist (1 U/ml) für 30 Minuten bei 4°C inkubiert. Anschließend werden die Zellen zweimal mit D'MEM/1% RSA gewaschen. Zur Detektion werden die Zellen mit Substrat (TMB) inkubiert.

Zur Isolierung des Rezeptors werden Hep G2-Zellen, die eine hohe Expression des Rezeptors zeigen, in T 150 Kulturflaschen gezüchtet und die sich gebildeten Zellschichten durch vorsichtiges Abschaben der Zellen geerntet. Zellen mehrerer Kulturgefäße werden vereint, um zu einer Gesamtmenge von 10⁹ Zellen zu gelangen. Anschließend werden die Zellen durch Inkubation mit 1% Triton X100 in PBS lysiert. Nach Zentrifugation bei 10.000 x g für 10 Minuten, wird der Zellüberstand separiert und das Präzipitat mit den Kern- und Zellmembranfraktionen in 1 M Natriumchlorid in PBS resuspendiert. Nach Zentrifugation bei 10.000 x g für 10 Minuten wird der Zellüberstand gesammelt und bei -20°C aufbewahrt. Diese Rezeptorpräparation kann für die Charakterisierung des Rezeptors in ELISA-Verfahren eingesetzt werden. 20 µg dieses rohen Rezeptorextraktes wird auf Flachboden-Mikrotiterplatten für 1 Stunde bei Raumtemperatur beschichtet. Nach Waschen mit PBS/0,1 % Tween®20 und Nachbeladen mit PBS/1% RSA wird der PSA-ACT-Komplex zugegeben und die Mischung 1 Stunde bei Raumtemperatur inkubiert. Die Platten werden gewaschen und danach mit einem Peroxidase-markierten anti-PSA Antikörper inkubiert. Die gebundene Peroxidase-Aktivität wird danach mit einem geeigneten Peroxidase-Farbsubstrat beispielsweise ABTS® nachgewiesen.

Zur weiteren Aufreinigung des rohen Rezeptorextraktes wird eine hydrophobe Chromatographie durchgeführt. Die Aktivität der einzelnen Fraktionen wird, wie oben beschrieben, mit einem ELISA nachgewiesen und die Fraktionen mit Rezeptoraktivität vereinigt. Anschließend wird der NH₂-Terminus des Rezeptormoleküls nach bekannten Methoden ansequenziert und die cDNA aus einer Genbank gefischt. Hierzu wird die aufgereinigte Rezeptorpreparation zunächst durch SDS-PAGE aufgetrennt. Die Proteinbanden werden ausgeschnitten und eluiert. Die Rezeptoraktivität der einzelnen Bande wird mittels eines Zell-ELISA durch Kompetition nachgewiesen. Der NH₂-Terminus der aktiven Bande wird anschließend mittels bekannter Methoden ansequenziert. Aus der Aminosäuresequenz wird die Nukleinsäuresequenz abgeleitet und entsprechende Oligoprimer synthetisiert. Ein zweiter degenerierter Primer aus der nicht translatierten Region vor der poly A-Region wird ebenfalls zur PCR Amplifikation genutzt, wobei Hep G2 DNA als Template genutzt wird. Mit Hilfe der amplifizierten Sequenz (ca 45 Nukleotide) werden positive Klone identifiziert und aufgereinigt. Die DNA wird durch Restriktionsanalyse und Southern Blots charakterisiert. Ein EcoRI-XbaI Fragment wird in Bluescript SK subkloniert. Die Sequenzanalyse des Klons und die Ableitung der Aminosäuresequenz beweist die Rezeptorspezifität. Mit Hilfe dieses Oligonukleotids wird eine DNA in einer cDNA Bank beispielsweise von foetaler Leber in λDR2 (Clontech Cat. No. HL 1151x) identifiziert, die mit der Rezeptorsequenz korreliert. Die identifizierten Klone werden aufgereinigt und mit Primem aus der Nähe der BAMHI-XbaI Region in λDR2 mit PCR analysiert. Die Phagensuspension wird als Template genutzt. Das Plasmid pDR2 wird isoliert und einer Restriktionsanalyse mit BAMHI und XbaI unterzogen. Das doppelsträngige DNA-Insert wird mit einem Autosequenzer (ABI 373) sequenziert nach Standardvorschriften für Farb-markierte Didesoxynukleosid-Triphosphatterminatoren und "walking primer" (Sanger et al., PNAS (1977) 74, 5463-5467).

Danach wird die Rezeptor-DNA isoliert und ein Expressionssystem konstruiert. Hierzu wird die cDNA, die den offenen Leserahmen für den Rezeptor enthält, in das Plasmid pSV15.JD.LL zwischen den Restriktionsstellen Cla I und Sal I kloniert. Der Vektor pSV15.JD.LL.SERC wird erhalten.

### a) Expression in E. coli

Das Plasmid enthält eine kurze Leader-Sequenz stromaufwärts des Rezeptorgens. Diese Leader-Sequenz erlaubt eine hohe Translationsrate und eine schnelle Aufreinigung. Nach Induktion des Tryptophan Promotors wird eine hohe intrazelluläre Produktion initiiert. Das Expressionsplasmid wird genutzt, um beispielsweise E. coli 44C6 mit Hilfe der CaCl₂-Hitzeschock-Methode nach Mandel et al., J. Mol. Biol. 53 (1970), 159-162 zu transformieren. Die so transformierten Zellen werden bei 37°C in LB Medium mit 50 µg/ml Carbenicillin bis zu einer optischen Dichte von 2-3 bei 600 nm wachsen gelassen. Die Suspension wird 20fach mit M9 Medium mit 0,49 % Casaminosäuren und 50 µg/ml Carbenicillin verdünnt. Für eine Stunde wird bei 30°C und Belüftung weiterkultiviert und Indolyl-3-acrylsäure bis zu einer Endkonzentration von 50 µg/ml zugegeben. Nach weiterem Kultivieren für 15 Stunden werden die Zellen geerntet.

### b) Expression in CHO oder andere Sängerzellen

Das Plasmid wird mit Not I linearisiert und CHO Zellen durch Elektroporation (Andreason, J. Tissue Culture Meth. 15 (1993), 56) transfektiert. Die Zellen werden in DHFR Selektionsmedium transferriert. Nach 2 Wochen werden individuelle Klone in 96er Mikrotiterplatten transferriert. Die Expression wird mittels kompetitivem ELISA gemessen.

### c) Die Expression kann ebenfalls im Baculovirus-System nach bekannten Methoden erfolgen

Anschließend an die Expression des Rezeptors wird dieser isoliert, aufgereinigt, charakterisiert und die Verwendung als Bindepartner in einem Immunoassay evaluiert. Hierzu wird der Kulturüberstand der transfizierten CHO Zellen gesammelt und auf eine Cibachromblue-Sepharose-Säule /100 Volumenanteile Zellüberstand/l Volumenanteil Säulenmaterial) aufgebracht. Die Säule wird mit 5 Volumenanteilen Applikationspuffer ohne Harnstoff und anschließend mit 10 mM Phosphatpuffer pH 7,4 mit 2 M Harnstoff (5 Volumenanteile) gewaschen. Der rekombinante Rezeptor wird mit 10 mM Phosphatpuffer pH 7,4, 2 M Harnstoff und 1 M NaCl eluiert. Die Rezeptor-enthaltenden Fraktionen werden auf eine Wheatgerm-Lectin-Säule aufgebracht. Nach Waschen mit 5 Volumentanteilen Applikationspuffer wird der Rezeptor mit 10 mM Phosphatpuffer pH 7,4, 2 M Harnstoff und 0,5 M N-Acetyl-D-Glucosamin eluiert.

Die vereinigten Rezeptor-haltigen Fraktionen werden auf 0,04 % C₁₂E₈ und 0,1 % TFA eingestellt. Mittels einer C4-Reversed-Phase-Säule werden die Proteine mit zwei konsekutiven linearen Acetonitrilgradienten (0-30 % und 30-60 % in 0,04 % C₁₂E₈ und 0,1 % TFA) separiert. Die Fraktionen werden mittels SDS-PAGE analysiert. Die Rezeptor-haltigen Fraktionen werden vereinigt und mit 2 Volumen 10 mM Natriumphosphatpuffer, pH 7,4 und 150 mM NaCl verdünnt und gegen 6 Volumen des Verdünnungspuffers in einer Ultrafiltrationskammer (Anschlußgröße 30.000) dialysiert und konzentriert.

Das Konzentrat wird auf 0,01 % Tween®80 eingestellt und im selben Puffer zur Entfernung von Aggregaten und Bruchstücken mittels Gelchromatographie aufgereinigt. Die rezeptorhaltigen Fraktionen (Nachweis durch SDS-PAGE) werden sterilfiltriert (Filter mit einem Porendurchmesser von 0,22 µm) und bei 4°C aufbewahrt.

Der in E. coli exprimierte Rezeptor wird folgendermaßen isoliert. Die E. coli Zellen werden in 10 Volumen Puffer (10 mM Tris-HCl, 5 mM EDTA, pH 8) homogenisiert und 30 Minuten bei 5000 x g zentrifugiert. Die Zellen werden in 10 Volumen Puffer (10 mM Tris-HCl, 5 mM EDTA, pH 8) aufgenommen, beispielsweise durch einen Mikrofluidizer passiert und zentrifugiert. Das Zellpellet wird bei -70°C eingefroren oder direkt weiterverwendet.

Das Zellpellet wird in 20 mM Tris-HCl, 8 M Guanidinhydrochlorid und 25 mM DTT,pH 8 resuspendiert und zur Solubilisierung der Rezeptormoleküle 12 Stunden bei 4°C gerührt. Nach Solubilisierung wird die Lösung 30 Minuten bei 30.000 x g zentrifugiert. Der klare Zellüberstand wird gewonnen und diese Lösung auf einer G200 Sephadexsäule (Gelchromatographiesäule) in 20 mM Na-Phosphatpuffer mit 10 mM DTT, pH 6 aufgereinigt. Die rezeptorhaltigen Fraktionen werden vereinigt (Nachweis des Proteins über SDS-PAGE). Diese Fraktionen werden, wie oben beschrieben, an einer C4-Reversed-Phase-Säule aufgetrennt. Die rezeptorhaltigen Fraktionen werden wiederum vereinigt und der Rezeptor renaturiert. Hierzu wird die Lösung mit 9 Volumen Renaturierungs-Puffer (5 mM EDTA, 2 % CHAPS-Detergenz 25 % Glycerin, 5 mM oxidiertem Glutathion und 1 mM reduziertem Glutathion, pH 8,3) verdünnt und 4 Tage bei 4°C dialysiert. Nach Renaturierung wird die Lösung auf 0,2 % TFA eingestellt, durch einen 0,45 µ Filter filtriert und auf 10 % Acetonitrile eingestellt. Danach folgt eine C4-Reversed-Phase-Säule nach obigen Angaben. Die rezeptorhaltigen Fraktionen werden gegen einen isotonischen Puffer (10 mM Na-Phosphatpuffer, 150 mM NaCl und 0,01 % Tween®80, pH 74,) dialysiert und bei 4°C aufbewahrt.

Der so hergestellte rekombinante Rezeptor kann als Bindepartner, entweder wandgebunden oder markiert, zur Bestimmung von PSA-Serpin-Komplexen in Immunoassays eingesetzt werden, wie oben beschrieben.

Die erfindungsgemäßen Tests zum Nachweis von PSA-ACT insbesondere die Einschritt-Tests eignen sich hervorragend zum Screenen einer großen Probenmenge, um einen Hinweis auf das Vorliegen eines Prostatacarcinoms zu erhalten. Es hat sich gezeigt, daß durch die Verwendung des erfindungsgemäßen Tests der Cut-Off im Vergleich zum bisher üblichen Cut-Off bei Tests zum Nachweis des totalen PSA erniedrigt werden kann. Auch in Bereichen unter den bisher üblichen Cut-Off-Werten kann mit dem erfindungsgemäßen Test noch eine vergleichsweise sichere Differenzierung zwischen Normalpatienten und Risikopatienten gefunden werden. Gleichzeitig werden aber vermehrt auch Patienten erfaßt, die ein Prostatacarcinom im Frühstadium besitzen, die bei den bisher üblichen Tests nicht erfaßt wurden. Die Cut-Off-Werte bei dem erfindungsgemäßen Test liegen deutlich niedriger als die entsprechenden Cut-Off-Werte für totales PSA. Der Cut-Off-Wert für PSA-ACT liegt bei ≤ 70 % des Cut-Off-Wertes für PSA, bevorzugt bei ≤ 60 % (in ng/ml). Bei gleicher Spezifität (95 % gegen BPH) liegt der Cut-Off-Wert für totales PSA beispielsweise bei 10,05 ng/m, der Cut- Off-Wert für PSA-ACT bei 5,70 ng/ml.

Bei den Patienten, die Werte über dem Cut-Off dieses erfindungsgemäßen Tests besitzten, wird zur Unterscheidung von benignen Erkrankungen und Prostatacarcinomen ein zweiter Test zum Nachweis des freien PSA vorgenommen. Solche Tests sind bereits seit 1985 erhältlich. Das Verhältnis von freiem PSA zu PSA-ACT wird bestimmt. Liegt das Verhältnis oberhalb von 0,1 bis 0,17 so ist dies ein starkes Indiz für das Vorliegen eines Carcinoms.

Die folgenden Beispiele sollen den Gegenstand der vorliegenden Erfindung verdeutlichen.

### Beispiel 1

### Herstellung monoklonaler Antikörper gegen PSA-ACT

### a) Immunisierung von Mäusen

12 Wochen alte, weibliche Balb/c-Mäuse werden mit 100 µg PSA-ACT (Fa. Centro, San Diego, Product Code CB 3075-01, Lot 50 10 70) zusammen mit dem Adjuvans CFA (Komplettes Freund'sches Adjuvans) intraperitoneal erstimmunisiert. Nach 6 Wochen folgen drei weitere Immunisierungen intraperitoneal in monatlichen Abständen. Dabei wird jeder Maus 100 µg PSA-ACT zusammen mit IFA (Inkomplettes Freund'sches Adjuvans) verabreicht. Anschließend erfolgen die letzten Immunisierungen intravenös mit je 100 µg PSA-ACT in PBS-Puffer am 3. und 2. Tag und letzten Tag vor der Fusion.

### b) Fusion und Klonierung

Die Fusion von Milzzellen der gemäß a) immunisierten Mäuse mit Myelomzellen erfolgt in Anlehnung an Galfré, Methods in Enzymology 73, 1981, 3. Dabei werden ca. 1 x 10⁸ Milzzellen der immunisierten Maus mit 2 x 10⁷ Myelomzellen (P3X63-Ag8-653, ATCC CRL1580) gemischt und abzentrifugiert (10 min bei 300 g und 4°C). Die Zellen werden dann einmal mit RPMI-1640-Medium ohne fötales Kälberserum (FKS) gewaschen und bei 400 g in einem 50 ml Spitzröhrchen erneut abzentrifugiert. Anschließend wird 1 ml PEG (Polyethylenglykol) (Molekulargewicht 4000, Merck, Darmstadt) dazugegeben, und durch Pipettieren gemischt. Nach 1 min im Wasserbad bei 37°C werden 5 ml RPMI 1640 ohne FKS zugetropft, durchmischt, auf 50 ml mit Medium (RPMI 1640 + 10% FKS) aufgefüllt und anschließend zentrifugiert. Die sedimentierten Zellen werden in BPMI 1640 Medium mit 10% FKS aufgenommen und in Hypoxanthin-Azaserin-Selektions-Medium (100 mmol/l Hypoxanthin, 1 µg/ml Azaserin in RPMI 1640 + 10% FKS) eingesät. Als Wachstumsfaktor wird dem Medium Interleukin 6 (100 U/ml) zugegeben.

Nach ca. 10 Tagen werden die Primärkulturen auf spezifische Antikörpersynthese getestet (siehe Beispiele 2). Primärkulturen, die mit PSA-ACT eine positive Reaktion zeigen und mit nicht komplexiertem PSA und nicht komplexiertem ACT sowie mit allen anderen Serumbestandteilen keine Kreuzreaktion aufweisen, werden mittels fluoreszenzaktiviertem Zellsorter in 96er-Zellkulturplatten kloniert. Hierbei wird dem Medium als Wachstumszusatz Interleukin-6 (100 U/ml) zugegeben.

Auf diese Weise wurden die in Tabelle I aufgeführten, hinterlegten Zellinien / Klone erhalten.

**Tabelle 1**

| Klon | IgG-Subklasse |
|---|---|
| | |
| 4. 3. 2 | Ig G1 |
| 6.13.64 | Ig G1 |
| 4.6.374 | Ig G1 |

### c) Immunglobulingewinnung aus den Zellkulturüberständen

Die erhaltenen Hybridomzellen werden mit einer Dichte von 1 x 10⁵ Zellen pro ml in RPMI 1640-Medium mit 10% FKS eingesät und 7 Tage lang in einem Fermenter (Fa. Thermodux, Wertheim/Main, Modell MCS-104XL, Best.-Nr. 144-050) vermehrt. In dem Kulturüberstand werden Konzentrationen von durchschnittlich 100 µg monoklonaler Antikörper je ml erreicht. Die Reinigung dieses Antikörpers aus dem Kulturüberstand erfolgt nach proteinchemisch üblichen Methoden (z.B. gemäß Methods in Enzymology 121 (1986), 587 - 695).

### Beispiel 2

### Screening-Test auf anti-PSA-ACT-Antikörper

Streptavidin-beschichtete MTP werden mit "Fang-Antikörpern" beschichtet, die PSA und PSA-ACT binden. Danach erfolgt die Inkubation mit dem Analyten PSA-ACT (bei a)) oder PSA (bei b)). Danach erfolgt die Inkubation mit dem zu testenden anti-PSA-ACT-Antikörper. Abschließend werden die gebundenen Antikörper mittels eines anti-Maus-IgG-POD in üblicher Weise durch Umsetzung eines Substrates nachgewiesen.

### a) Bestimmung der Spezifität mit PSA-ACT

Um die Spezifität der Antikörper im Kulturüberstand der Hybridomzellen zu bestimmen, werden mit rekombinantem Streptavidin beschichtete MTP (Fa. MicroCoat, Penzberg, Best.-Nr. 12-K 96 N, Charge MC 289) mit 10 µg/ml biotinyliertem Fab-Fragment des monoklonalen Antikörpers 1 oder des monoklonalen Antikörpers 2 (beide monoklonalen Antikörper erkennen nicht komplexiertes PSA sowie PSA im Komplex) in PBS plus 0,5 % Crotein C beschichtet (100 µl pro well, 10 min Inkubation bei Raumtemperatur unter Schütteln) und anschließend 3 x mit 0,9 % NaCl / 0,05% Tween 20 gewaschen.

Danach wird mit 100 ng/ml PSA-ACT (Fa. Scripps, San Diego, Kat. Nr. P 0624, Charge 66 15 64 oder Fa. Centro, San Diego, Kat. Nr. CB 30 75 01, Charge 50 10 70) gelöst in PBS plus 0,5 % Crotein C inkubiert (100 µl pro well, 1 h unter Schütteln bei Raumtemperatur). Anschließend wird 3 x mit 0,9 % NaCl / 0,05% Tween 20 gewaschen.

Im nächsten Schritt wird 100 µl der zu untersuchenden Antikörper-Lösung ( im Kulturüberstand ) in ein beschichtetes well gegeben und 1 Stunde bei Raumtemperatur unter Schütteln inkubiert. Nach 3-maligem Waschen mit 0,9 % Natriumchlorid / 0,05% Tween 20 wird zum Nachweis von gebundenem Antikörper aus der Probe jeweils 100 µl eines POD-markierten Fab-Fragments eines polyklonalen Antikörpers aus dem Schaf gegen Maus-Fcγ (Boehringer Mannheim GmbH, Ident.-Nr. 1431323, ensprechend 25 mU / ml) zugegeben, 1 Stunde bei Raumtemperatur unter Schütteln inkubiert und anschließend 3 x mit 0,9% Natriumchlorid / 0,05% Tween® 20 gewaschen.

Schließlich werden jeweils 100 µl / well ABTS®-Lösung (Boehringer Mannheim GmbH, Kat.-Nr. 1204521 und 1204530) zugegeben und nach 30 min bei Raumtemperatur die Extinktion bei 405/492 nm in einem MR700 Microplate Reader der Firma Dynatech gemessen.

### b) Bestimmung der Reaktivität / Kreuzreaktion mit PSA

Um die Reaktivität / Kreuzreaktion mit PSA zu bestimmen wird im unter a) beschriebenen Test mit nicht komplexiertem PSA, statt mit PSA-ACT inkubiert.

Dazu werden mit rekombinantem Streptavidin beschichtete MTP (Fa. MicroCoat, Penzberg, Best.-Nr. 12-K 96 N, Charge MC 289) mit 10 µg/ml biotinyliertem Fab-Fragments des monoklonalen Antikörpers 1 oder des monoklonalen Antikörpers 2 (beide monoklonalen Antikörper erkennen nicht komplexiertes PSA sowie PSA im Komplex) in PBS plus 0,5 % Crotein C beschichtet (100 µl pro well, 10 min Inkubation bei Raumtemperatur unter Schütteln) und anschließend 3 x mit 0,9 % NaCl / 0,05% Tween®20 gewaschen.

Danach wird mit 50 ng/ml PSA (Fa. Scripps, San Diego, Kat. Nr. P 0714, Charge 98 43 64 ) gelöst in PBS plus 0,5 % Crotein C inkubiert (100 µl pro well, 1 Stunde unter Schütteln bei Raumtemperatur). Anschließend wird 3 x mit 0,9 % NaCl / 0,05% Tween® 20 gewaschen.

Im nächsten Schritt wird 100 µl der zu untersuchenden Antikörper-Lösung (im Kulturüberstand ) in ein beschichtetes well gegeben und 1 Stunde bei Raumtemperatur unter Schütteln inkubiert. Nach 3-maligem Waschen mit 0,9 % Natriumchlorid / 0,05% Tween® 20 wird zum Nachweis von gebundenem Antikörper aus der Probe jeweils 100 µl eines POD-markierten Fab-Fragments eines polyklonalen Antikörpers aus dem Schaf gegen Maus-Fcγ (Boehringer Mannheim GmbH, Ident.-Nr. 1431323, ensprechend 25 mU / ml) zugegeben, 1 Stunde bei Raumtemperatur unter Schütteln inkubiert und anschließend 3 x mit 0,9% Natriumchlorid / 0,05% Tween 20 gewaschen.

Schließlich werden jeweils 100 µl / well ABTS®-Lösung(Boehringer Mannheim GmbH, Kat.-Nr. 1204521 und 1204530) zugegeben und nach 30 min bei Raumtemperatur die Extinktion bei 405/492 nm in einem MR700 Microplate Reader der Firma Dynatech gemessen.

### c) Bestimmung der Reaktivität mit ACT

Um die Reaktivität mit ACT zu bestimmen wird im unter a) beschriebenen Test der zu untersuchende Antikörper mit ACT im Überschuß vorinkubiert. Bleibt das Meßsignal unverändert hoch, liegt keine Kreuzreaktion vor, ist das Meßsignal erniedrigt, liegt eine Kreuzreaktion vor.

Dazu werden mit rekombinantem Streptavidin beschichtete MTP (Fa. MicroCoat, Penzberg, Best.-Nr. 12-K 96 N, Charge MC 289) mit 10 µg/ml biotinyliertem Fab-Fragments des monoklonalen Antikörpers 1 oder des monoklonalen Antikörpers 2 (beide monoklonalen Antikörper erkennen nicht komplexiertes PSA sowie PSA im Komplex) in PBS plus 0,5 % Crotein C beschichtet (100 µl pro well, 10 min Inkubation bei Raumtemperatur unter Schütteln) und anschließend 3 x mit 0,9 % NaCl / 0,05% Tween® 20 gewaschen.

Danach wird mit 100 ng/ml PSA-ACT (Fa. Scripps oder Fa. Centro) gelöst in PBS plus 0,5 % Crotein C inkubiert (100 µl pro well, 1 Stunde unter Schütteln bei Raumtemperatur). Anschließend wird 3 x mit 0,9 % NaCl / 0,05% Tween 20 gewaschen.

Der auf Kreuzreaktion zu prüfende Antikörper wird mit einer Konzentrationsreihe von 0, 10 µg, 50 µg, 100 µg / ml ACT (Fa. Athens, Athen, Best. Nr. 16-16-012400, Charge AX 9501) vorinkubiert. Die Vorinkubation erfolgt in unbeschichteten 96er-MTP-wells für 1 h bei Raumtemperatur unter Schütteln.

Von dieser Lösung (Antikörper + ACT im Überschuß) wird im nächsten Schritt 100 µl in ein beschichtetes well gegeben und 1 h bei Raumtemperatur unter Schütteln inkubiert. Nach 3-maligem Waschen mit 0,9 % Natriumchlorid / 0,05% Tween 20 wird zum Nachweis von gebundenem Antikörper aus der Probe jeweils 100 µl eines POD-markierten Fab-Fragments eines polyklonalen Antikörpers aus dem Schaf gegen Maus-Fcy (Boehringer Mannheim GmbH, Ident.-Nr. 1431323 ensprechend 25 mU / ml) zugegeben, 1 Stunde bei Raumtemperatur unter Schütteln inkubiert und anschließend 3 x mit 0,9% Natriumchlorid / 0,05% Tween® 20 gewaschen.

Schließlich werden jeweils 100 µl / well ABTS®-Lösung (Boehringer Mannheim GmbH, Kat.-Nr. 1204521 und 1204530) zugegeben und nach 30 min bei Raumtemperatur die Extinktion bei 405/492 nm in einem MR700 Microplate Reader der Firma Dynatech gemessen.

### d) Bestimmung der Reaktivität mit anderen Serumbestandteilen

Um die Reaktivität mit anderen Serumkomponenten zu bestimmen wird im unter a) beschriebenen Test der zu untersuchende Antikörper mit humanen, weiblichen Seren vorinkubiert. Bleibt das Meßsignal unverändert hoch, liegt keine Kreuzreaktion vor, ist das Meßsignal erniedrigt, liegt eine Kreuzreaktion vor.

Dazu wurden mit rekombinantem Streptavidin beschichtete MTP (Fa. MicroCoat, Penzberg, Best.-Nr. 12-K 96 N, Charge MC 289) mit 10 µg/ml biotinyliertem Fab-Fragments des monoklonalen Antikörpers 1 oder des monoklonalen Antikörpers 2 (beide monoklonalen Antikörper erkennen nicht komplexiertes PSA sowie PSA im Komplex) in PBS plus 0,5 % Crotein C beschichtet (100 µl pro well, 10 min Inkubation bei Raumtemperatur unter Schütteln) und anschließend 3 x mit 0,9 % NaCl / 0,05% Tween® 20 gewaschen.

Danach wird mit 100 ng/ml PSA-ACT (Fa. Scripps oder Fa. Centro) gelöst in PBS plus 0,5 % Crotein C inkubiert (100 µl pro well, 1 Stunde unter Schütteln bei Raumtemperatur). Anschließend wird 3 x mit 0,9 % NaCl / 0,05% Tween® 20 gewaschen.

Der auf Kreuzreaktion zu prüfende Antikörper wird mit einer Konzentrationsreihe (1:1 bis 1:10 ) von humanen, weiblichen Seren (einer Mischung von vier PSA-negativen Spenderinnen) vorinkubiert. Die Vorinkubation erfolgt in unbeschichteten 96er-MTP-wells für 1 h bei Raumtemperatur unter Schütteln.

Von dieser Lösung (Antikörper + weibliche Humanseren) wird im nächsten Schritt 100 µl in ein beschichtetes well gegeben und 1 Stunde bei Raumtemperatur unter Schütteln inkubiert. Nach 3-maligem Waschen mit 0,9 % Natriumchlorid / 0,05% Tween® 20 wird zum Nachweis von gebundenem Antikörper aus der Probe jeweils 100 µl eines POD-markierten Fab-Fragments eines polyklonalen Antikörpers aus dem Schaf gegen Maus-Fcγ (Boehringer Mannheim GmbH, Ident.-Nr. 1431323 ensprechend 25 mU / ml) zugegeben, 1 Stunde bei Raumtemperatur unter Schütteln inkubiert und anschließend 3 x mit 0,9% Natriumchlorid / 0,05% Tween® 20 gewaschen.

Schließlich werden jeweils 100 µl / well ABTS®-Lösung (Boehringer Mannheim GmbH, Kat.-Nr. 1204521 und 1204530) zugegeben und nach 30 min bei Raumtemperatur die Extinktion bei 405/492 nm in einem MR700 Microplate Reader der Firma Dynatech gemessen.

Alle hinterlegten monoklonalen Antikörper zeigten eine starke Reaktivität zu PSA-ACT. Mit diesen Testmethoden konnte bei keinem der hinterlegten monoklonalen Antikörper eine klinisch relevante Reaktivität zu PSA, ACT und anderen Serumbestandteilen nachgewiesen werden. Die hinterlegten monoklonalen Antikörper wurden bei einem Screening von mehreren tausend monoklonalen Antikörper gefunden, von denen ca. 70% stark mit ACT und ca. 30% stark mit PSA kreuzreagierten.

### Beispiel 3

### Bestimmung der Affinitätkonstanten sowie der Geschwindigkeitskonstanten der Assoziation und Dissoziation der produzierten Antikörper

Die Bestimmung der Affinitätkonstanten sowie der Geschwindigkeitskonstanten der Assoziation und Dissoziation der produzierten Antikörper erfolgte mit BIAcore® der Firma Pharmacia Biosensor (BIA steht für Biospezifische Interaktions Analyse). Das Messprinzip beruht auf der "Surface Plasmon Resonance". Die Messung wird auf einem Biosensor, dem sog. Sensorchip durchgeführt. Hierbei wird auf die mit carboxmethyliertem Dextran beschichtete Oberfläche eines Sensorchips (CM5, Pharmacia Biosensor) ein polyklonaler Kaninchen-Antikörper gegen den Fcy-Teil von Maus-IgG über seine Aminogruppen kovalent gekoppelt. Über diesen Sensorchip wird eine Lösung des zu bestimmenden Antikörpers geleitet, wobei der Antikörper durch nichtkovalente Wechselwirkungskräfte an den immobilisierten Fangantikörper gebunden wird. Im folgenden wird das zu untersuchende Antigen über den Sensorchip geleitet, welches dann durch ebenfalls nichtkovalente Wechselwirkungskräfte an den über den Fangantikörper immobilisierten Antikörper gebunden wird.

Durch die Bindung der einzelnen Komponenten erhöht sich die Massendichte auf der Oberfläche des Sensorchips, die vom Gerät in ein proportionales Messignal umgewandelt wird. Aus der zeitlichen Änderung des Signals, dem Sensorgramm, lassen sich die Geschwindigkeitskonstanten der Assoziation und Dissoziation und daraus die Affinitäts-konstante berechnen.

Die Antikörper-Antigen-Komplexe lassen sich ohne Beeinträchtigung der an die Oberfläche gebundenen Fangantikörper mit einfachen Mitteln wieder ablösen, so daß an demselben Sensorchip weitere Bindungsexperimente unter identischen Randbedingungen durchgeführt werden können.

Zur Kopplung des Fangantikörpers an den Sensorchip (CM5, Pharmacia Biosensor) wird eine Lösung des Antikörpers (BIA certified Rabbit anti Mouse Fcγ, Pharmacia Biosensor) mit einer Konzentration von 60 µg/ml in 10 mM Natriumacetat-Puffer pH 5.0 mit einer Flußrate von 5 µl/min über den zuvor mit NHS/EDC aktivierten Sensorchip geleitet.

Danach werden die Antikörper zugegeben, so daß eine Zunahme der an die Oberbäche gebundenen Masse von mindestens 600 Resonanzeinheiten erfolgt. Die Bindung der Antigene an die Antikörper werden bei einer Flußrate von 10 µl/min verfolgt und die Geschwindigkeitskonstanten der Assoziation und der Dissoziation für die Bindung an die Antikörper aus den Sensorgrammen mit Hilfe einer Software des Herstellers (BIAevaluation 2.1, Pharmacia Biosensor) berechnet. Die Affinitätskonstante berechnet sich nach Ka = kon/koff. Die so ermittelten Werte der erfindungsgemäßen Antikörper mit PSA-ACT, Chymotrypsin-ACT und CathepsinG-ACT als Antigene sind in der folgenden Tabelle 2 zusammengefaßt. Sowohl mit nicht komplexiertem PSA, Chymotrysin, Cathepsin G als auch mit freiem humanem ACT als Antigen kann keine Bindung nachgewiesen werden, d.h. die Affinitätskonstante zu diesen Verbindungen ist kleiner als 10⁵ l/mol.

**Tabelle 2**

| | PSA-ACT | | | Chymotrypsin-ACT | | | CathepsinG-ACT | | |
|---|---|---|---|---|---|---|---|---|---|
| Klon | kon | koff | Ka | kon | koff | Ka | kon | koff | Ka |
| | l/mol*s | l/s | l/mol | l/mol*s | l/s | l/mol | l/mol*s | l/s | l/mol |
| 4.6.374 | 1*10⁵ | 1*10⁻⁵ | 1*10¹⁰ | 1*10⁵ | 3*10⁻⁴ | 3*10⁸ | 1*10⁵ | 5*10⁻⁵ | 2*10⁸ |
| 4.3.2 | 3*10⁴ | 9*10⁻⁴ | 4*10⁷ | 8*10⁴ | 3*10⁻³ | 3*10⁷ | 1*10⁵ | 1*10⁻³ | 9*10⁷ |
| 6.13.64 | 5*10⁴ | 2*10⁻³ | 2*10⁷ | 1*10⁵ | 3*10⁻³ | 3*10⁷ | 1*10⁵ | 2*10⁻³ | 8*10⁷ |
| 4.1.358 | 5*10⁴ | 6*10⁻³ | 9*10⁶ | 5*10⁴ | 6*10⁻³ | 9*10⁶ | 8*10⁴ | 2*10⁻³ | 5*10⁷ |
| 4.9.358 | 5*10⁴ | 6*10⁻³ | 9*10⁶ | 5*10⁴ | 6*10⁻³ | 9*10⁶ | 8*10⁴ | 2*10⁻³ | 4*10⁷ |

### Beispiel 4

### Screening zum Nachweis eines Verdachts auf Prostatacarcinom

### a) Screeningtest

Es wurden Kollektive aus 276 gesunden Männern, 456 Patienten mit benigner Prostatahyperplasie (BPH) und 348 Patienten mit nachgewiesenem Prostatacarcinom (PCa) untersucht.

Im Serum wurde das gesamte PSA mit dem Enzymun-Test PSA bestimmt. Außerdem wurde PSA-ACT gemessen, indem der Nachweisantikörper des Enzymun-Test® PSA durch den für PSA-ACT spezifischen Antikörper 4.6.374 (nach dessen POD-Markierung) ausgetauscht und sonst nach der Vorschrift des Beipackzettels verfahren wurde.

### b) Ergebnis

Die Verteilung der PSA-Werte in den drei Kollektiven ist aus Tabelle 3 ersichtlich. Unter dem üblichen Cut-off-Wert von 4 ng PSA / mL lagen 90 % der Gesunden, 38 % der BPH-Patienten und 16 % der Carcinompatienten. Probanden, deren PSA-Wert über dem Cut-off liegt, werden weiteren Untersuchungen zugeführt; während diejenigen, deren PSA-Konzentration unter dem Entscheidungswert liegen, als Prostata-gesund betrachtet werden.

Verwendet man statt PSA 4 ng/ml die Grenze von PSA-ACT bei 3 nglml, so liegen wieder 90% der Gesunden unterhalb dieses Wertes, während aber in diesem Fall 3 zusätzliche Prostatacarcinompatienten richtig erkannt werden (siehe Tabelle 4).

Durch den Austausch des Screeningparameters wurde also bei derselben Spezifität gegenüber dem Normalkollektiv eine höhere Sensitivität in der Carcinomgruppe erreicht.

**Tabelle 3**

| **Cut-off: PSA 4 ng/ml** | | | |
|---|---|---|---|
| | Gesunde | BPH | PCa |
| Geamtzahl der Werte | 276 | 456 | 348 |
| Anzahl der Werte im Bereich | | | |
| PSA ≤ 4 ng/mL | 249 | 174 | 54 |
| PSA > 4 ng/mL | 27 | 282 | 294 |
| | | | |
| Anteil (%) der Werte im Bereich | | | |
| PSA ≤ 4 ng/mL | 90,2 | 38,2 | 15,5 |
| PSA > 4 ng/mL | 9,8 | 61,8 | 84,5 |

**Tabelle 4**

| **Cut-off: PSA-ACT 3 ng/ml** | | | |
|---|---|---|---|
| | Gesunde | BPH | PCa |
| Gesamtzahl der Werte | 276 | 456 | 348 |
| Anzahl der Werte im Bereich | | | |
| PSA-ACT ≤ 3 ng/mL | 248 | 158 | 51 |
| PSA-ACT > 3 ng/mL | 28 | 298 | 297 |
| | | | |
| Anteil (%) der Werte im Bereich | | | |
| PSA-ACT ≤ 3 ng/mL | 89,9 | 34,6 | 14,7 |
| PSA-ACT > 3 ng/mL | 10,1 | 65,4 | 85,3 |
| | | | |

### Beispiel 5

### Immunoassay zur Bestimmung von PSA-ACT

### a) Durchführung des Immunoassays am ES 300 (Boehringer Mannheim GmbH)

50 µl Serumprobe bzw. PSA-ACT-Standard wurden für 1 Stunde mit 700 µl Reagenz 1 (40 mmol/l Natriumphosphatpuffer, pH 7,4 / 0,2 % (w/v) Rinderserumalbumin / 1,2 µg/ml biotinylierter monoklonaler anti-PSA-Antikörper M10, Fab Fragment, in Streptavidin-beschichteten Enzymun®-Universaltubes inkubiert und anschließend gewaschen.

Danach wurden 700 µl Reagenz 2 (40 mmol/l Natriumphosphatpuffer, pH 7,4 / 0,2 % (w/v) Rinderserumalbumin / 0,1 % Rinder-IgG / monoklonaler anti-PSA-ACT-Antikörper, Klon 4.6.374, (Fab)₂'-POD-Konjugat, 95 mU/ml) zugefügt und nach 30 min Inkubation gewaschen.

Die Farbentwicklung erfolgte mit 700 µl Substratlösung (1 mg/ml ABTS® / 0,5 mg/ml Natriumperborat/Citrat-/Phosphatpuffer) während 30 min und wurde bei 422 nm im Photometer gemessen.

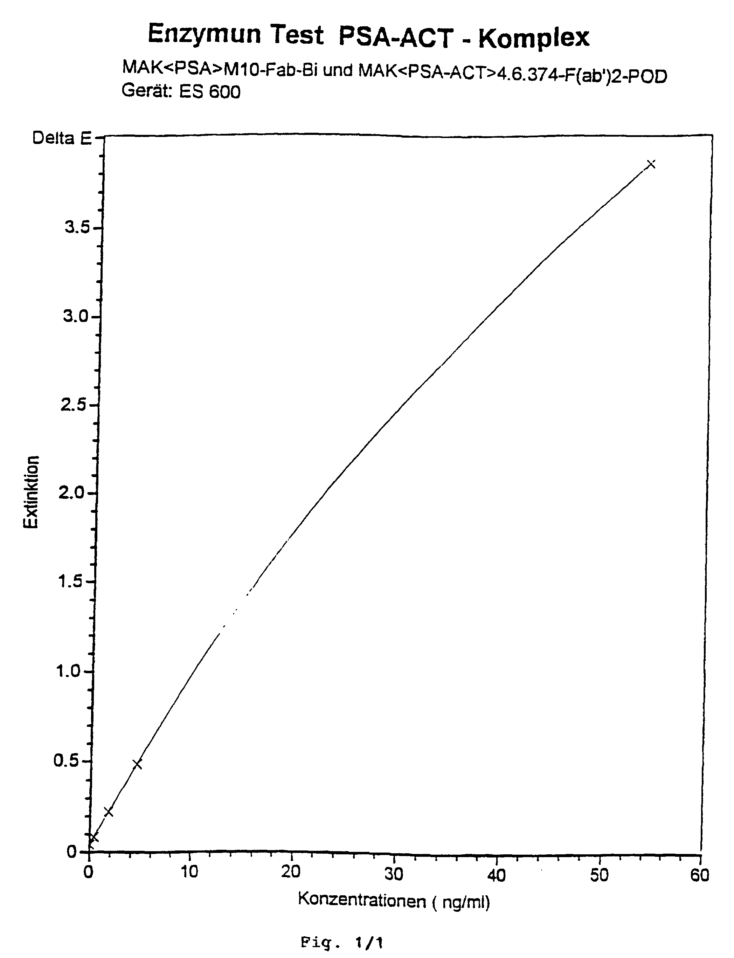

Fig. 1 zeigt eine typische Eichkurve.

### b) Relevante Kreuzreaktionen im Test

In dem unter a) beschriebenen Testsystem wurde als Probe ein Frauenserum verwendet, in dem kein endogenes PSA meßbar war. Diesem Serum wurde in separaten Aliquots gereinigtes ACT (Fa. Serva) bzw. Chymotrypsin-ACT-Komplex (jeweils 50 µg/ml) zugesetzt. Dieselben Zusätze wurden einem Humanserum mit erhöhtem endogenen PSA-Gehalt zugefügt.

Desweiteren wurde statt Serum eine Lösung von 22 ng/ml freiem PSA in Phosphatpuffer zum Test verwendet.

Die in diesen Proben gemessenen PSA-ACT-Gehalte sind aus Tabelle 5 ersichtlich. Die gemessene Kreuzreaktion für PSA und ACT im Test war in allen Fällen <0,003 % und keiner der Zusätze führte zu einem für die Beurteilung der Probe verfälschenden PSA-ACT-Ergebnis.

### c) Messung von Seren gesunder Frauen und von Frauen mit verschiedenen Entzündungen

Mit dem unter a) beschriebenen Test wurden die PSA-ACT-Gehalte in 40 Seren gesunder Frauen und in 18 Seren von Patientinnen mit verschiedenen Entzündungen gemessen. In der ersten Gruppe war der PSA-ACT-Spiegel im Mittel 0,01 ng/ml (± 0,03 ng/ml Standardabweichung), während bei der Entzündungsgruppe im Mittel 0,03 (± 0,04) ng PSA-ACT/ml gefunden wurde.

Dies belegt, daß andere ACT-Komplexe, z.B. das bei Entzündungen erhöhte Cathepsin G-ACT, oder andere natürlich vorkommende ACT-Protease-Komplexe, nicht zu Störungen im Test führen.

**Tabelle 5**

| **Probe** | **PSA-ACT gemessen [ng/ml]** | **Kreuzreaktion im Test** |
|---|---|---|
| Humanserum, PSA-frei | | |
| ohne Zusatz | 0 | |
| + ACT [50 µg/ml] | 0 | 0 |
| + Chymotrypsin-ACT [50 µg/ml] | 0,13 | 0,0003 % |
| Humanserum, unbehandelt | | |
| ohne Zusatz | 41,7 | |
| + ACT [50 µg/ml] | 43,0 | 0,0026 % |
| + Chymotrypsin-ACT [50 µg/ml] | 42,0 | 0,0020 % |
| Freies PSA in Puffer [22 ng/ml] | 0 | 0 |

### Beispiel 6

### Sensitivität und Spezifität der Identifikation von Prostatacarcinom

In einem Kollektiv von 48 Seren von Patienten mit benigner Prostatahyperplasie (BPH) und von 45 Patienten mit Prostatacarcinom wurde t PSA und fPSA mit den jeweiligen Enzymun®Tests und PSA-ACT nach Beispiel 5 bestimmt. Der PSA-Gehalt lag in beiden Gruppen unter 20 ng/ml. Um eine Aussage zur Diskriminierung des Prostatacarcinoms gegenüber BPH zu erhalten, wurde eine ROC-Auswertung durchgeführt (Zweig, M.H., Clin.Chem. 39 (1993) 561-577:"Receiver-Operating Characteristic (ROC) Plots: A Fundamental Tool in Clinical Medicine") und an den jeweiligen Kurven die korrespondierenden Werte für Sensitivität und Spezifität abgelesen. Außerdem wurde die Fläche unter der ROC-Kurve berechnet, die ein Maß für das Diskriminanzvermögen eines Parameters darstellt, zwischen dem entsprechenden Tumor- und benignem Erkrankungskollektiv zu unterscheiden. Je größer diese Fläche ist, desto wahrscheinlicher ist die korrekte Zuordnung einer unbekannten Probe.

**Tabelle 6:**

| Ergebnis der ROC-Auswertung. | | |
|---|---|---|
| **Parameter** | **Fläche unter Kurve** | **Bei Spezifität 95% ergibt sich Sensitivität (%)** |
| t PSA | 0,709 | 29 |
| PSA-ACT | 0,748 | 40 |
| fPSA | 0,438 | 9 |
| ROC-Auswertung im Bereich 0 - 20 ng PSA/ml | | |
| BPH: n=48, PCa: n=45 | | |

Die Sensivität des Erkennens von Prostatacarcinom gegenüber der benignen Prostatahyperplasie war bei derselben Spezifität von 95% bei Messung des PSA-ACT 40 % gegenüber 29 % bei t PSA. Auch die Fläche unter der Kurve erhöhte sich von 0,709 auf 0,748 wenn man statt t PSA das PSA-ACT betrachtet.

Die Messung von freiem PSA als singulärem Parameter erscheint wegen der niedrigen Sensitivität nicht sinnvoll. Die Fläche unter der fPSA-Kurve war ebenfalls deutlich niedriger als diejenigen der beiden übrigen Parameter.

### Beispiel 7

### Korrelation von berechneten und gemessenen PSA-ACT-Werten

Unter der Annahme, daß sich das immunologisch erfaßbare Gesamt-PSA aus den beiden Hauptkomponenten fPSA und PSA-ACT zusammensetzt, kann nach Messung der beiden ersten Parameter der dritte berechnet und dieses Ergebnis mit den gemessenen Werten des 3. Parameters verglichen werden. Nach Bestimmung von t PSA und fPSA mit den jeweiligen Enzymun®Testen wurde deren Differenz berechnet und die Korrelation zu dem nach Beispiel 5 gemessenen PSA-ACT ermittelt.

**Tabelle 7:**

| Korrelation von gemessenem und berechnetem PSA-ACT-Gehalt | | | | |
|---|---|---|---|---|
| Parameter 1 | Parameter 2 | Kollektiv | Anzahl Proben | Korrelation r |
| PSA-ACT | tPSA-fPSA | | | |
| | | alle Proben | 264 | 0.974 |
| (= Y- Wert) | (= X- Wert) | Kontrollen, männ1. | 70 | 0.965 |
| | | | | |
| | | BPH | 51 | 0.947 |
| | | PCa | 60 | 0.967 |
| | | Andere Erkrankungen männl. Patienten | 83 | 0.993 |

Über alle 264 Werte stimmte der gemessene und berechnete PSA-ACT-Gehalt mit r=0,974 sehr gut überein und war in den Einzelgruppen überall größer als 0,946. Dies zeigt, daß der PSA-ACT-Test mit dem neuen monoklonalen Antiköper ein sehr plausibles Ergebnis erzielt.

## Patentansprüche

1. Monoldonale Antikörper gegen einen Komplex aus humanem ACT und einer Serinprotease, **dadurch gekennzeichnet, daß** sie im wesentlichen keine Kreuzreaktivität mit nicht komplexiertem humanem ACT und freien Serinproteasen nachweisbar besitzen.

2. Monoklonale Antikörper nach Anspruch 1, **dadurch gekennzeichnet, daß** sie eine höhere Affinität und Spezifität zu PSA-ACT als zu anderen Serinproteasen-ACT-Komplexen aufweist.

3. Monoklonale Antikörper nach einem der Ansprüche 1-2, **dadurch gekennzeichnet, daß** sie eine mindestens 10fach höhere Affinität zu PSA-ACT als zu Chymotrypsin-ACT und Cathepsin-G-ACT aufweisen.

4. Monoklonale Antikörper nach einem der Ansprüche 1-3, **dadurch gekennzeichnet, daß** sie keine Kreuzreaktivität zu anderen Bestandteilen in humanem Serum aufweisen.

5. Monoklonale Antikörper nach einem der Ansprüche 1-4, **dadurch gekennzeichnet, daß** sie keine Kreuzreaktivität zu anderen Bestandteilen von Proben humanen Ursprungs wie Plasma, Serum, Blut, Seminalflüssigkeit, Prostata-Flüssigkeit, Seminal-Vesikel-Flüssigkeit, Speichel, Liquor, Frauen-Milch, Zysten, Gewebe-Homogenate, Gewebeschnitte und Biopsie-Material aufweisen.

6. Monoklonale Antikörper nach einem der Ansprüche 1-5, **dadurch gekennzeichnet, daß** sie eine Affinität zu PSA-ACT von mehr als 10⁷l/mol besitzen.

7. Monoklonale Antikörper nach einem der Ansprüche 1-6, **dadurch gekennzeichnet, daß** sie eine Affinität zu PSA von mehr als 10⁹ l/mol besitzen.

8. Monoklonale Antikörper nach Anspruch 1, **dadurch gekennzeichnet, daß** sie von einer der Zellinien MAK<PSA-ACT>M 4.6.374, MAK<PSA-ACT>M 4.3.2 oder MAK<PSA-ACT>M 6.13.64 mit den Hinterlegungsnummern DSM ACC 2281, DSM ACC 2283 und DSM ACC 2282 produziert werden.

9. Antikörper nach Anspruch 1, **dadurch gekennzeichnet, daß** sie in äquivalenter Weise binden, wie die monoklonalen Antikörper, die von den Zellinien MAK<PSA-ACT>M 4.6.374, MAK<PSA-ACT>M 4.3.2 und MAK<PSA-ACT>M 6.13.64 mit den Hinterlegungsnummern DSM ACC 2281, DSM ACC 2283 und DSM ACC 2282 produziert werden.

10. Monoklonale Antikörper nach einem der Ansprüche 1-9, **dadurch gekennzeichnet, daß** sie vom IgG1-Typ sind.

11. Monoklonale Antikörper nach einem der Ansprüche 1-10, **dadurch gekennzeichnet, daß** sie durch Immunisierung eines geeigneten Versuchstieres, anschließender Fusion der Milzzellen des immunisierten Tieres mit Myelomzellen erhalten wurden.

12. Verwendung der monoklonalen Antikörper gemäß einem der Ansprüche 1-11, in einem diagnostischen Test zum Nachweis von PSA-ACT.

13. Verfahren zur Bestimmung eines Komplexes aus ACT und Serinproteasen durch Inkubation der Probe mit mindestens einem monoklonalen Antikörper nach einem der Ansprüche 1-11.

14. Verfahren nach Anspruch 13, **dadurch gekennzeichnet, daß** der Komplex PSA-ACT bestimmt wird.

15. Verfahren nach Anspruch 14, **dadurch gekennzeichnet, daß** außerdem ein Antikörper gegen PSA eingesetzt wird.

16. Screeningtest zum Nachweis des Vorliegens eines Prostatacarcinoms mittels eines Verfahrens gemäß Anspruch 14, **dadurch gekennzeichnet, daß** zum Ausschluß der nicht malignen Proben ein Cut-Off von 2,2 - 3,2 ng PSA ACT/ml gesetzt wird.

17. Screeningtest nach Anspruch 16, **dadurch gekennzeichnet, daß** der Test zum Nachweis des Komplexes aus humanem ACT und PSA ohne Waschschritt zum Entfernen von überschüssigem ACT durchgeführt wird.

## Claims

1. Monoclonal antibodies against a complex of human ACT and a serine protease, wherein they have essentially no detectable cross-reactivity with non-complexed human ACT and free serine proteases.

2. Monoclonal antibodies as claimed in claim 1, wherein they have a higher affinity and specificity for PSA-ACT than for other serine protease-ACT complexes.

3. Monoclonal antibodies as claimed in one of the claims 1 to 2, wherein they have an at least 10-fold higher affinity for PSA-ACT than for chymotrypsin-ACT and cathepsin-G-ACT.

4. Monoclonal antibodies as claimed in one of the claims 1 to 3, wherein they have no cross-reactivity with other components in human serum.

5. Monoclonal antibodies as claimed in one of the claims 1 to 4, wherein they have no cross-reactivity with other components of samples of human origin such as plasma, serum, blood, seminal fluid, prostate fluid, seminal vesicle fluid, saliva, liquor, human milk, cysts, tissue homogenates, tissue sections and biopsy material.

6. Monoclonal antibodies as claimed in one of the claims 1 to 5, wherein they have an affinity for PSA-ACT of more than 10⁷ l/mol.

7. Monoclonal antibodies as claimed in one of the claims 1 to 6, wherein they have an affinity for PSA of more than 10⁹ l/mol.

8. Monoclonal antibodies as claimed in claim 1, wherein they are produced by one of the cell lines MAK<PSA-ACT>M 4.6.374, MAK<PSA-ACT>M 4.3.2 or MAK<PSA-ACT>M 6.13.64 with the depository numbers DSM ACC 2281, DSM ACC 2283 and DSM ACC 2282.

9. Antibodies as claimed in claim 1, wherein they bind in an equivalent manner to the monoclonal antibodies which are produced by the cell lines MAK<PSA-ACT>M 4.6.374, MAK<PSA-ACT>M 4.3.2 and MAK<PSA-ACT>M 6.13.64 with the depository numbers DSM ACC 2281, DSM ACC 2283 and DSM ACC 2282.

10. Monoclonal antibodies as claimed in one of the claims 1 to 9, wherein they are of the IgG1 type.

11. Monoclonal antibodies as claimed in one of the claims 1 to 10, wherein they have been obtained by immunizing a suitable experimental animal and subsequently fusing the spleen cells of the immunized animal with myeloma cells.

12. Use of the monoclonal antibodies as claimed in one of the claims 1 to 11 in a diagnostic test for the detection of PSA-ACT.

13. Method for the determination of a complex of ACT and serine proteases by incubating the sample with at least one monoclonal antibody as claimed in one of the claims 1 to 11.

14. Method as claimed in claim 13, wherein the complex PSA-ACT is determined.

15. Method as claimed in claim 14, wherein in addition an antibody against PSA is used.

16. Screening test to detect the presence of a prostate carcinoma using a method as claimed in claim 14, wherein the cut-off is set at 2.2 - 3.2 ng PSA-ACT/ml to exclude non-malignant samples.

17. Screening test as claimed in claim 16, wherein the test for detecting the complex of human ACT and PSA is carried out without a wash step to remove excess ACT.

## Revendications

1. Anticorps monoclonaux contre un complexe de l'ACT humaine et d'une sérineprotéase, **caractérisés en ce qu'**ils ne possèdent essentiellement aucune réactivité croisée décelables avec de l'ACT humaine non complexée et avec des sérineprotéases libres.

2. Anticorps monoclonaux selon la revendication 1, **caractérisés en ce qu'**ils présentent une affinité et une spécificité vis-à-vis du complexe PSA-ACT supérieure à celles vis-à-vis de complexes d'autres sérineprotéases-ACT.

3. Anticorps monoclonaux selon l'une quelconque des revendications 1 à 2, **caractérisés en ce qu'**ils présentent une affinité vis-à-vis du PSA-ACT au moins 10 fois supérieure à celles vis-à-vis de la chymotrypsine-ACT et de la cathepsine-G-ACT.

4. Anticorps monoclonaux selon l'une quelconque des revendications 1 à 3, **caractérisés en ce qu'**ils ne présentent pas de réactivité croisée avec d'autres constituants du sérum humain.

5. Anticorps monoclonaux selon l'une quelconque des revendications 1 à 4, **caractérisés en ce qu'**ils ne présentent pas de réactivité croisée avec d'autres constituants d'échantillons d'origine humaine tels que le plasma, le sérum, le sang, le sperme, le liquide prostatique, le liquide des vésicules séminales, le crachat, le liquide céphalo-rachidien, le lait maternel humain, les kystes, les homogénats tissulaires, les coupes tissulaires et les matières obtenues par biopsie.

6. Anticorps monoclonaux selon l'une quelconque des revendications 1 à 5, **caractérisés en ce qu'**ils possèdent une affinité vis-à-vis du PSA-ACT de plus de 10⁷ l/mole.

7. Anticorps monoclonaux selon l'une quelconque des revendications 1 à 6, **caractérisés en ce qu'**ils possèdent une affinité vis-à-vis du PSA de plus de 10⁹ l/mole.

8. Anticorps monoclonaux selon la revendication 1, **caractérisés en ce qu'**ils sont produits à partir d'une des lignées cellulaires MAK<PSA-ACT>M 4.6.374, MAK<PSA-ACT>M 4.3.2 ou MAK<PSA-ACT>M 6.13.64 portant les numéros de dépôt DSM ACC 2281, DSM ACC 2283 et DSM ACC 2282.

9. Anticorps monoclonaux selon la revendication 1, **caractérisés en ce qu'**ils se lient de manière équivalente à celle des anticorps monoclonaux qui sont produits à partir des lignées cellulaires MAK<PSA-ACT>M 4.6.374, MAK<PSA-ACT>M 4.3.2 ou MAK<PSA-ACT>M 6.13.64 portant les numéros de dépôt DSM ACC 2281, DSM ACC 2283 et DSM ACC 2282.

10. Anticorps monoclonaux selon l'une quelconque des revendications 1 à 9, **caractérisés en ce qu'**ils sont de type IgG1.

11. Anticorps monoclonaux selon l'une quelconque des revendications 1 à 10, **caractérisés en ce qu'**on les obtient par immunisation d'un animal d'essai approprié, et par fusion ultérieure des cellules de la rate de l'animal immunisé avec des cellules de myélome.

12. Utilisation des anticorps monoclonaux selon l'une quelconque des revendications 1 à 11, dans un essai diagnostique pour le décèlement du PSA-ACT.

13. Procédé pour la détermination d'un complexe de ACT et de sérineprotéases par incubation de l'échantillon avec au moins un anticorps monoclonal selon l'une quelconque des revendications 1 à 11.

14. Procédé selon la revendication 13, **caractérisé en ce qu'**on détermine le complexe PSA-ACT.

15. Procédé selon la revendication 14, **caractérisé en ce qu'**on met en oeuvre en outre un anticorps contre le PSA.

16. Essai de dépistage pour le décèlement de la présence d'un carcinome de la prostate à l'aide d'un procédé selon la revendication 14, **caractérisé en ce que**, afin d'exclure les échantillons non malins, on règle une limite de 2,2 - 3,2 ng de PSA-ACT/ml.

17. Essai de dépistage selon la revendication 16, **caractérisé en ce qu'**on effectue l'essai pour le décèlement du complexe de l'ACT humaine et de la PSA, en l'absence d'une étape de lavage pour éliminer l'ACT en excès.
